# EUROPÄISCHE PATENTSCHRIFT

(11) **EP 2 745 797 B1**
(45) Veröffentlichungstag und Bekanntmachung des Hinweises auf die Patenterteilung: **25.10.2017**
(21) Anmeldenummer: 12198873.7
(22) Anmeldetag: 21.12.2012
(51) Int. Cl.: A61C 3/12, A61B 17/32, A61C 3/03

(54) **Ein durch Schall antreibbares Schneidwerkzeug**
An acoustically driveable cutting tool
Outil de coupe à entraînement sonore

(43) Veröffentlichungstag der Anmeldung: 25.06.2014
(73) Patentinhaber: W & H Dentalwerk Bürmoos GmbH, 5111 Bürmoos (AT)
(72) Erfinder: Brandstätter, Andreas, 5120 St. Pantaleon (AT); Lette, Andreas, 4050 Traun (AT)
(74) Vertreter: Benda, Ralf

(56) Entgegenhaltungen:
- EP-A1- 0 456 470
- EP-A1- 0 637 433
- EP-A1- 1 736 107
- WO-A1-2009/157621
- KR-B1- 100 999 106
- US-A- 5 180 363

## Beschreibung

Die vorliegende Erfindung betrifft ein durch Schall antreibbares, medizinisches, insbesondere dentales, Schneidwerkzeug mit einem lamellenförmigen Arbeitsabschnitt.

Ein derartiges Werkzeug ist aus der Patentanmeldung EP 0 456 470 A1 bekannt. Dieses Werkzeug umfasst ein Anschlussteil zur Verbindung des Schneidwerkzeugs mit einer Schallquelle, einen lamellenförmigen Arbeitsabschnitt und einen den Anschlussteil mit dem lamellenförmigen Arbeitsabschnitt verbindenden Schaft, wobei sich eine Flüssigkeitsleitung durch den Schaft bis zu dem lamellenförmigen Arbeitsabschnitt erstreckt und wobei der lamellenförmige Arbeitsabschnitt zwei im Wesentlichen parallel zueinander angeordnete Seitenflächen und eine die zwei Seitenflächen verbindende Umfangskante aufweist. An der Umfangskante sind Schneidabschnitte vorgesehen.

Die Patentanmeldung WO 2009/157621 A1 offenbart ein durch Schall antreibbares, dentales Schneidwerkzeug für den maxilliaren Sinuslift, dessen Arbeitsbereich mehrere Zähne mit jeweils mehrere geneigten Schneidflächen aufweist.

Der vorllegenden Erfindung liegt die Aufgabe zugrunde, ein durch Schall antreibbares, medizinisches, insbesondere dentales, Schneidwerkzeug mit einer höheren Schneidleistung und einer verbesserten Handhabung für den Anwender zu schaffen.

Diese Aufgabe wird gemäß der vorliegenden Erfindung durch ein durch Schall antreibbares, medizinisches, insbesondere dentales, Schneidwerkzeug, nach Anspruch 1 und eine medizinische Behandlungvorrichtung nach Anspruch 9 gelöst. Die Erfindung betrifft auch ein Verfahren gemäß Anspruch 10 zur Herstellung des erfindungsgemäßen Schneidwerkzeugs. Aufgrund der Neigung zumindest einer Schneidfläche eines Zahns des Schneidwerkzeugs ausgehend von einer Seitenflächen des lamellenförmigen Arbeitsabschnitts in Richtung der anderen Seitenflächen des lamellenförmigen Arbeitsabschnitts, (4) und / oder aufgrund der größeren Breite der Seitenflächen des lamellenförmigen, Arbeitsabschnitts zumindest in einem Teilbereich des lamellenförmigen Arbeitsabschnitts. Insbesondere in dem ersten Abschnitt mit den Schneidelementen, in Bezug auf den Außendurchmesser des Schafts konnte in praktischen Versuchen eine merklich verbesserte Schneidleitung des Schneidwerkzeugs beobachtet werden, insbesondere in Hartgewebe, Zahngewebe oder Zahnschmelz. Des Weiteren bewirkt der zweite Abschnitt ohne Schneidelemente eine bessere Führung des Schneidwerkzeugs, insbesondere bei tieferem Eindringen in das zu schneidende Gewebe, insbesondere in Hartgewebe.

Vorzugsweise ist zumindest ein Teil des Werkzeugs aus Metall gefertigt, insbesondere aus Stahl. Zur besseren Handhabung weist das Werkzeug, insbesondere der Schaft, vorzugsweise zumindest eine Biegung auf. Vorzugsweise ist an der Oberfläche des Werkzeugs, insbesondere des Anschlussteils, eine Schlüsselfläche zum Anbringen eines Instruments vorgesehen, welches das Verbinden des Werkzeugs mit der Schall- oder Ultraschallquelle unterstützt. Vorzugsweise ist eine Schwingachse oder Sonotrode vorgesehen, welche die Schall- oder Ultraschallquelle mit dem Werkzeug verbindet. Vorzugsweise ist das Werkzeug lösbar mit der Schall- oder Ultraschallquelle und / oder mit der Schwingachse oder Sonotrode verbindbar.

Vorzugsweise umfasst das Anschlussteil ein Verbindungselement zur, insbesondere lösbaren, Verbindung des Werkzeugs mit der Schall- oder Ultraschallquelle. Das Verbindungselement ist zum Beispiel als Gewinde ausgebildet. Vorzugsweise ist das Anschlussteil hohl oder hülsenartig ausgebildet. Vorzugsweise umfasst das Anschlussteil einen Innenraum, insbesondere eine zylindrische Innenbohrung mit einer Innenwand. Vorzugsweise ist das Verbindungselement, insbesondere das Gewinde, im Innenraum oder an der Innenwand des Anschlussteils vorgesehen. Vorzugsweise ist das Anschlussteil im Wesentlichen zylindrisch oder hohlzylindrisch ausgebildet. Vorzugsweise ist das Anschlussteil länglich ausgebildet und erstreckt sich entlang einer Mittelachse. Vorzugsweise bildet der Innenraum des Anschlussteils einen Teil der Flüssigkeitsleitung des Werkzeugs oder der Innenraum des Anschlussteils ist zur Leitung einer Flüssigkeit ausgebildet. Vorzugsweise ist der Innendurchmesser des Innenraums des Anschlussteils größer als der Innendurchmesser zumindest eines Abschnitts der Flüssigkeitsleitung in dem Schaft und / oder in dem Arbeitsabschnitt. Vorzugsweise ist der Außendurchmesser des Anschlussteils größer als der Außendurchmesser zumindest eines Abschnitts des Schafts. Vorzugsweise sind der Anschlussteil und der Schaft über einen sich in Richtung des Schafts verjüngenden Verbindungsabschnitt miteinander verbunden.

Vorzugsweise ist der Schaft gebogen ausgebildet, er kann jedoch auch im Wesentlichen gerade sein. Vorzugsweise ist der Schaft länglich ausgebildet und erstreckt sich entlang einer Mittelachse. Vorzugsweise verringert sich der Außendurchmesser des Schafts zumindest geringfügig in Richtung des Arbeitsabschnitts. Vorzugsweise umfasst der Schaft eine, insbesondere zentrische, Bohrung. Besonders bevorzugt ist die Bohrung des Schafts Teil der Flüssigkeitsleitung des Werkzeugs. Vorzugsweise ist die Bohrung des Schafts mit dem Innenraum des Anschlussteils verbunden, insbesondere flüssigkeitsübertragen verbunden. Vorzugsweise ist die Bohrung des Schafts mit zumindest einer Öffnung, insbesondere flüssigkeitsübertragen, verbunden, so dass Flüssigkeit aus der Bohrung auf zumindest eine Seitenfläche des lamellenartigen Arbeitsabschnitts abgebbar ist. Vorzugsweise ist der Außendurchmesser des Schafts geringer als 3,0 mm.

Vorzugsweise umfasst die Flüssigkeitsleitung mehrere Abschnitte, zum Beispiel den Innenraum des Anschlussteils, die Bohrung des Schafts oder zumindest eine Öffnung zur Abgabe von Flüssigkeit auf zumindest eine Seitenfläche des lamellenartigen Arbeitsabschnitts. Vorzugsweise endet die Flüssigkeitsleitung in dieser zumindest einen Öffnung. Vorzugsweise weist die Flüssigkeitsleitung unterschiedliche Innendurchmesser auf. Vorzugsweise ist der Innendurchmesser der Flüssigkeitsleitung zumindest in einem Abschnitt des Anschlussteils größer als in einem Teil des Schafts und / oder des Arbeitsabschnitts. Vorzugsweise ist die Flüssigkeitsleitung ausgebildet ist, an beiden Seitenflächen und / oder auf beide Seitenflächen des lamellenförmigen Arbeitsabschnitts und / oder in Richtung der Behandlungsstelle Flüssigkeit abzugeben.

Gemäß einem Ausführungsbeispiel ist der erste Abschnitt mit den Schneidelementen am Vorderende des lamellenförmigen Arbeitsabschnitts und der zweite Abschnitt ohne Schneidelemente zwischen dem Schaft und dem ersten Abschnitt mit den Schneidelementen vorgesehen ist.

Vorzugsweise erstreckt sich der lamellenförmigen Arbeitsabschnitt entlang einer Längsachse. Vorzugsweise umfasst der lamellenförmigen Arbeitsabschnitt einen Teilbereich mit einer größten Breite. Vorzugsweise ist zumindest in diesem Teilbereich mit der größten Breite die Breite der Seitenflächen des lamellenförmigen Arbeitsabschnitts größer ist als der Außendurchmesser des Schafts. Vorzugsweise weist der lamellenförmigen Arbeitsabschnitt oder die Umfangskante in dem Teilbereich mit der größten Breite zumindest einen Apex auf. Vorzugsweise verjüngt sich die Breite des lamellenförmigen Arbeitsabschnitts von dem Apex in Richtung des Vorderendes des lamellenförmigen Arbeitsabschnitts. Vorzugsweise sind zumindest einige der Schneidelemente des lamellenförmigen Arbeitsabschnitts auf dem sich in Richtung des Vorderendes verjüngenden Abschnitt angeordnet. Insbesondere weisen zumindest einige dieser Schneidelemente freie Enden, zum Beispiel Spitzen auf, die im Wesentlichen auf einer ersten Geraden angeordnet sind, wobei die erste Gerade gewinkelt zur Längsachse des lamellenförmigen Arbeitsabschnitts angeordnet ist. Die Breite des lamellenförmigen Arbeitsabschnitts verjüngt sich von dem Apex in Richtung des Schafts. Zumindest einige der Schneidelemente des lamellenförmigen Arbeitsabschnitts sind auf dem sich in Richtung des Schafts verjüngenden Abschnitt angeordnet. Insbesondere weisen zumindest einige dieser Schneidelemente freie Enden, zum Beispiel Spitzen auf, die im Wesentlichen auf einer zweiten Geraden angeordnet sind, wobei die zweite Gerade gewinkelt zur Längsachse des lamellenförmigen Arbeitsabschnitts angeordnet ist. Vorzugsweise sind die erste Gerade und die zweite Gerade gewinkelt zueinander angeordnet.

Vorzugsweise ist zumindest ein Abschnitt des Außenrands des lamellenförmigen Arbeitsabschnitts oder der Umfangskante, insbesondere zumindest ein Teil des ersten Abschnitts mit den Schneidelementen, zwischen dem Apex und dem Vorderende des Schneidwerkzeugs konkav ausgebildet. Vorzugsweise verjüngt sich der lamellenförmigen Arbeitsabschnitt oder die Umfangskante, insbesondere zumindest ein Teil des zweiten Abschnitts ohne Schneidelemente, von dem Apex in Richtung des Schafts. Besonders bevorzugt ist zumindest ein Abschnitt des Außenrands des lamellenförmigen Arbeitsabschnitts oder der Umfangskante, insbesondere zumindest ein Teil des zweiten Abschnitts ohne Schneidelemente, zwischen dem Apex und dem Schaft des Schneidwerkzeugs konvex ausgebildet.

Vorzugsweise beträgt die Dicke oder Stärke des lamellenförmigen Arbeitsabschnitts in etwa 0,2 mm - 0,4 mm, bevorzugt in etwa 0,25 mm - 0,3 mm. Vorzugsweise beträgt die Härte (Vickershärte) des lamellenförmigen Arbeitsabschnitts oder des Materials des lamellenförmigen Arbeitsabschnitts in etwa 495 - 550 HV 10, bevorzugt in etwa 505 - 530 HV 10, besonders bevorzugt in etwa 510 HV 10. Beide Merkmale bewirken in vorteilhafter Weise, insbesondere in Kombination, dass das Schneidwerkzeug oder der lamellenförmige Arbeitsabschnitt in Hartgewebe, insbesondere in Zahngewebe oder Zahnschmelz, eindringen oder dieses schneiden kann.

Zur weiteren Verbesserung der Schneidleistung weist das Schneidwerkzeug zumindest eines der folgenden Merkmale auf:
- die Schneidelemente des ersten Abschnitts weisen mehrere Zähne auf;
- an dem Schneidelement des ersten Abschnitts sind unterschiedliche Zähne vorgesehen, die sich zum Beispiel in zumindest einem der folgenden Merkmale unterschieden: Der Größe, der Länge, der Breite oder der Form des Zahns, dem Neigungswinkel zumindest einer Zahnflanke, dem Neigungswinkel oder der Neigungsrichtung des gesamten Zahns, der Form eines freien Endes des Zahns;
- die Zähne haben eine Basis, welche sie mit dem lamellenförmigen Arbeitsabschnitt verbindet, eine Spitze und eine sich von der Basis zur Spitze erstreckende Höhe, wobei an dem Vorderende des ersten Abschnitts mit den Schneidelementen (oder des lamellenförmigen Arbeitsabschnitts oder der Umfangskante) mehrere erste Zähne und an einem daran anschließenden Teilbereich zwischen dem Vorderende des ersten Abschnitts und dem zweiten Abschnitt ohne Schneidelemente mehrere zweite Zähne vorgesehen sind, wobei die ersten Zähne kleiner sind als die zweiten Zähne oder wobei die Höhe der ersten Zähne kleiner ist als die Höhe der zweiten Zähne;
- an einem an den zweiten Abschnitt ohne Schneidelemente anschließenden Teilbereich des ersten Abschnitts mit den Schneidelementen sind mehrere dritte Zähne und an einem daran anschließenden Teilbereich des ersten Abschnitts mehrere zweite Zähne vorgesehen, wobei die dritten Zähne kleiner sind als die zweiten Zähne oder wobei die Höhe der dritten Zähne kleiner ist als die Höhe der zweiten Zähne.

Gemäß einem Ausführungsbeispiel ist der lamellenförmige Arbeitsabschnitt über einen Verbindungsabschnitt mit der Schaft verbunden, wobei der Verbindungsabschnitt eine in Richtung des Schafts zunehmende Außenabmessung aufweist und wobei die Seitenflächen des lamellenförmigen Arbeitsabschnitts sich in dem Verbindungsabschnitt fortsetzen. Insbesondere nimmt die Außenabmessung des Verbindungsabschnitts an den sich in dem Verbindungsabschnitt fortsetzenden Seitenflächen des lamellenförmigen Arbeitsabschnitts zu. Vorzugsweise ist die Breite der sich in dem Verbindungsabschnitt fortsetzenden Seitenflächen des lamellenförmigen Arbeitsabschnitts zumindest in einem Teilbereich des Verbindungsabschnitts größer als der Außendurchmesser des Schafts.

Eine medizinische, insbesondere dentale, Behandlungsvorrichtung, ist definiert durch ein durch Schall antreibbares, medizinisches, insbesondere dentales, Schneidwerkzeug und durch eine Schallquelle, wobei das Schneidwerkzeug mit der Schallquelle verbindbar und durch die Schallquelle in Schwingung versetzbar ist. Das durch Schall antreibbare, medizinische, insbesondere dentale, Schneidwerkzeug, umfasst: Ein Anschlussteil zur Verbindung des Schneidwerkzeugs mit einer Schallquelle, einen lamellenförmigen Arbeitsabschnitt und einen den Anschlussteil mit dem lamellenförmigen Arbeitsabschnitt verbindenden Schaft, wobei sich eine Flüssigkeitsleitung durch den Schaft bis zu dem lamellenförmigen Arbeitsabschnitt erstreckt, wobei der lamellenförmige Arbeitsabschnitt zwei im Wesentlichen parallel zueinander angeordnete Seitenflächen und eine die zwei Seitenflächen verbindende Umfangskante aufweist, wobei die Umfangskante des lamellenförmigen Arbeitsabschnitts einen ersten Abschnitt mit Schneidelementen und einen zweiten Abschnitt ohne Schneidelemente aufweist und wobei die Breite der Seitenflächen des lamellenförmigen Arbeitsabschnitts zumindest in einem Teilbereich des lamellenförmigen Arbeitsabschnitts, insbesondere in dem ersten Abschnitt mit den Schneidelementen, größer ist als der Außendurchmesser des Schafts.

Vorzugsweise umfasst die Schallquelle, insbesondere die Ultraschallquelle, einen elektrisch betreibbaren Schwingungserzeuger, zum Beispiel einen piezokeramischen oder einen magnetostriktiven Schwingungserzeuger. Vorzugsweise ist eine Schwingachse oder Sonotrode vorgesehen, welche die Schall- oder Ultraschallquelle schwingungsübertragend mit dem Werkzeug verbindet. Vorzugsweise ist zumindest eine Teil der Schwingachse oder Sonotrode hohl ausgebildet, insbesondere zur Leitung einer Flüssigkeit. Vorzugsweise sind das Werkzeug und die Schwingachse oder Sonotrode flüssigkeitsübertragend miteinander verbunden.

Vorzugsweise umfasst die Flüssigkeitsquelle eine sterile Flüssigkeit, insbesondere eine physiologische Kochsalzlösung. Vorzugsweise umfasst die Flüssigkeitsquelle eine Pumpe und / oder eine Leitungsvorrichtung, zum Beispiel einen Schlauch oder eine Leitung, zur Verbindung mit dem Werkzeug.

Vorzugsweise umfasst die Behandlungsvorrichtung ein Handstück, das lösbar mit dem Werkzeug verbindbar ist. Vorzugsweise ist in dem Handstück zumindest eines der folgenden Elemente aufgenommen: Zumindest ein Teil der Schallquelle, insbesondere der Ultraschallquelle, die Schwingachse oder Sonotrode, zumindest ein Teil der Flüssigkeitsquelle, insbesondere zumindest einen Teil der Leitungsvorrichtung.

Ein Verfahren zur Herstellung eines durch Schall antreibbaren, medizinischen, insbesondere dentalen, Schneidwerkzeugs ist dadurch definiert, dass ein lamellenförmiger Arbeitsabschnitt mit zwei im Wesentlichen parallel zueinander angeordnete Seitenflächen und eine die zwei Seitenflächen verbindende Umfangskante mit einem ersten Abschnitt mit Schneidelementen und einem zweiten Abschnitt ohne Schneidelemente derart geformt wird, dass die Breite der Seitenflächen des lamellenförmigen Arbeitsabschnitts zumindest in einem Teilbereich des lamellenförmigen Arbeitsabschnitts, insbesondere in dem ersten Abschnitt mit den Schneidelementen, größer ist als der Außendurchmesser des Schafts des Schneidwerkzeugs. Vorzugsweise werden bei dem Verfahren als Schneidelemente mehrere Zähne geformt, vorzugsweise durch Laserbearbeitung oder Laserschneiden. Vorzugsweise werden bei dem Verfahren an dem Vorderende des ersten Abschnitts mit den Schneidelementen (oder des lamellenförmigen Arbeitsabschnitts oder der Umfangskante) mehrere erste Zähne und an einem daran anschließenden Teilbereich zwischen dem Vorderende des ersten Abschnitts und dem zweiten Abschnitt ohne Schneidelemente mehrere zweite Zähne geformt, wobei die ersten Zähne kleiner sind als die zweiten Zähne oder wobei die Höhe der ersten Zähne kleiner ist als die Höhe der zweiten Zähne. Besonders bevorzugt werden bei dem Verfahren an einem an den zweiten Abschnitt ohne Schneidelemente anschließenden Teilbereich des ersten Abschnitts mit den Schneidelementen mehrere dritte Zähne und an einem daran anschließenden Teilbereich des ersten Abschnitts mehrere zweite Zähne geformt, wobei die dritten Zähne kleiner sind als die zweiten Zähne oder wobei die Höhe der dritten Zähne kleiner ist als die Höhe der zweiten Zähne.

Die Erfindung wird nachfolgend anhand bevorzugter Ausführungsbeispiele und Bezug nehmend auf die beigefügten Zeichnungen erläutert. Es zeigt die
Figur 1 eine Außenansicht eines Ausführungsbeispiels eines durch Schall antreibbaren, medizinischen, insbesondere dentalen, Schneidwerkzeugs.
Figur 2 einen Längsschnitt durch das Schneidwerkzeug der Figur 1.
Figur 3 eine vergrößerte Darstellung des Arbeitsabschnitts des Schneidwerkzeug der Figur 1.
Figur 4 eine Vorderansicht des Schneidwerkzeugs der Figur 1.
Figur 5 eine medizinische, insbesondere dentale, Behandlungsvorrichtung, mit einem durch Schall antreibbaren, medizinischen, insbesondere dentalen, Schneidwerkzeug.
Figur 6 ein Ausführungsbeispiel eines Zahns des durch Schall antreibbaren, medizinischen, insbesondere dentalen, Schneidwerkzeugs.

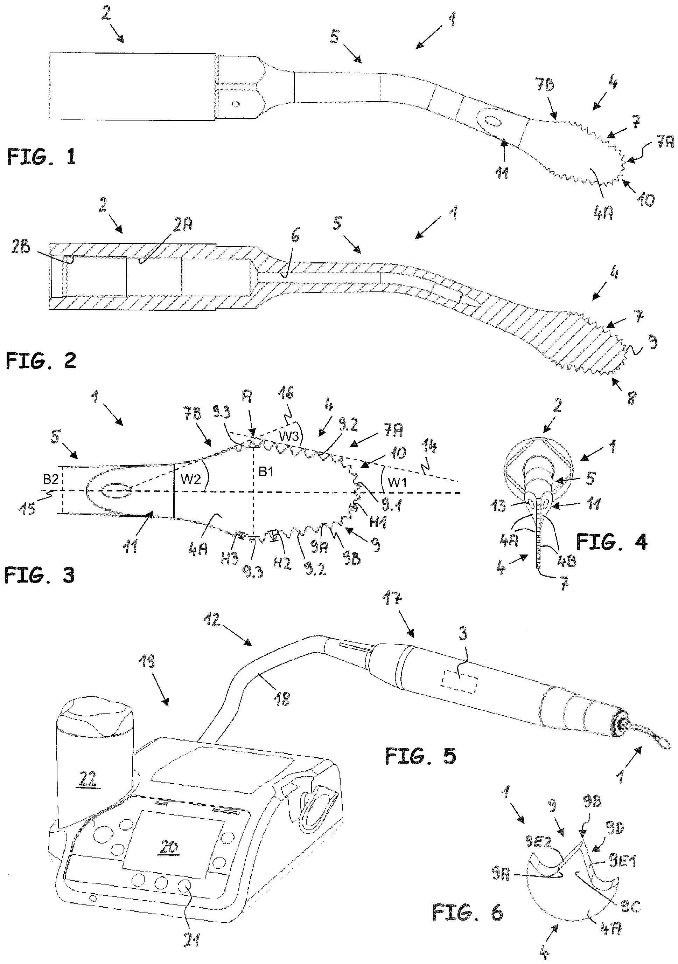

Das in den Figuren 1 - 4 dargestellte durch Schall antreibbare, medizinische, insbesondere dentale, Schneidwerkzeug 1 umfasst ein Anschlussteil 2 zur Verbindung des Schneidwerkzeugs 1 mit einer Schallquelle 3, einen lamellenförmigen Arbeitsabschnitt 4 und einen den Anschlussteil 2 mit dem lamellenförmigen Arbeitsabschnitt 4 verbindenden Schaft 5. Eine Flüssigkeitsleitung 6 erstreckt sich durch das Anschlussteil 2, den Schaft 5 bis zu dem lamellenförmigen Arbeitsabschnitt 4.

Das Anschlussteil 2 weist eine zylindrischen Innenbohrung 2A auf, wobei an der Wand der Innenbohrung 2A ein Verbindungselement 2B zur, insbesondere lösbaren, Verbindung des Schneidwerkzeugs 1 mit der Schallquelle 3 vorgesehen ist. Das Verbindungselement 2B ist zum Beispiel als Gewinde oder Innengewinde ausgebildet.

Der lamellenförmige Arbeitsabschnitt 4 weist zwei im Wesentlichen parallel zueinander angeordnete Seitenflächen 4A, 4B und eine die zwei Seitenflächen 4A, 4B verbindende Umfangskante 7 auf. Die Umfangskante 7 weist einen ersten Abschnitt 7A mit Schneidelementen 8 und einen zweiten Abschnitt 7B ohne Schneidelemente auf. Als Schneidelemente 8 des ersten Abschnitts 7A sind mehrere Zähne 9, 9.1, 9.2, 9.3 vorgesehen.

Der lamellenförmige Arbeitsabschnitt 4 ist über einen Verbindungsabschnitt 11 mit der Schaft 5 verbunden, wobei der Verbindungsabschnitt 11 eine in Richtung des Schafts 5 zunehmende Außenabmessung aufweist und wobei die Seitenflächen 4A, 4B des lamellenförmigen Arbeitsabschnitts 4 sich in dem Verbindungsabschnitt 11 fortsetzen. Die sich in dem Verbindungsabschnitt 11 fortsetzenden Seitenflächen 4A, 4B entfernen sich somit von der Ebene, in welcher sich der lamellenförmige Arbeitsabschnitt 4 erstreckt (siehe insbesondere Figur 4). An dem Verbindungsabschnitt 11 sind mehrere, zum Beispiel zwei, Öffnungen 13 vorgesehen, die mit der Flüssigkeitsleitung 6 verbunden sind oder welche die Enden der Flüssigkeitsleitung 6 bilden. Durch diese Öffnungen 13 ist Flüssigkeit aus der Flüssigkeitsleitung 6 auf den lamellenförmigen Arbeitsabschnitt 4 und / oder die Behandlungsstelle abgebbar. Bevorzugt ist an jeder sich in dem Verbindungsabschnitt 11 fortsetzenden Seitenflächen 4A, 4B zumindest eine Öffnung 13 vorgesehen, so dass insbesondere an beiden Seitenflächen 4A, 4B des lamellenförmigen Arbeitsabschnitts 4 Flüssigkeit abgebbar ist. Vorzugsweise verzweigt sich die Flüssigkeitsleitung 6 in dem Schaft 5 oder nahe der Öffnungen 13, so dass jeder Öffnung 13 eine eigene Zweigleitung zugeordnet ist.

Die Figur 3 zeigt insbesondere den detaillierten Aufbau des lamellenförmigen Arbeitsabschnitts 4: Die Breite B1 der Seitenflächen 4A, 4B des lamellenförmigen Arbeitsabschnitts 4 ist zumindest in einem Teilbereich des lamellenförmigen Arbeitsabschnitts 4, insbesondere in dem ersten Abschnitt 7A mit den Schneidelementen 8 oder im Bereich der größten Zähne 9.2, größer als der Außendurchmesser B2 des Schafts 5.

An dem lamellenförmigen Arbeitsabschnitt 4 oder an dem ersten Abschnitt 7A sind mehrere Zähne 9 vorgesehen, die sich in zumindest einem Merkmal voneinander unterscheiden, zum Beispiel in ihrer Größe, in ihrer Höhe oder in ihrer Breite. Die Zähne 9 weisen eine Basis 9A, welche sie mit dem lamellenförmigen Arbeitsabschnitt 4 verbindet, eine Spitze 9B und eine sich von der Basis 9A zur Spitze 9B erstreckende Höhe H1, H2, H3 auf. An dem Vorderende 10 des ersten Abschnitts 7A mit den Schneidelementen 8 oder des lamellenförmigen Arbeitsabschnitts 4 sind mehrere erste Zähne 9.1 und an einem daran anschließenden Teilbereich zwischen dem Vorderende 10 und dem zweiten Abschnitt 7B ohne Schneidelemente mehrere zweite Zähne 9.2 vorgesehen. Die ersten Zähne 9.1 sind kleiner als die zweiten Zähne 9.2 oder die Höhe H1 der ersten Zähne 9.1 ist kleiner als die Höhe H2 der zweiten Zähne 9.2.

Bevorzugt sind an einem an den zweiten Abschnitt 7B ohne Schneidelemente anschließenden Teilbereich des ersten Abschnitts 7A mit den Schneidelementen 8 mehrere dritte Zähne 9.3 vorgesehen, wobei die dritten Zähne 9.3 kleiner sind als die im Vorstehenden beschriebenen zweiten Zähne 9.2 oder wobei die Höhe H3 der dritten Zähne 9.3 kleiner ist als die Höhe H2 der zweiten Zähne 9.2. Bevorzugt sind die zweiten Zähne 9.2 und die dritten Zähne 9.3 oder ihre Höhen H2, H3 in etwa oder exakt gleich groß.

Der lamellenförmigen Arbeitsabschnitt 4 oder die Umfangskante 7 weisen in dem Teilbereich mit der größten Breite B1 zumindest einen Apex A auf. Die Breite des lamellenförmigen Arbeitsabschnitts 4 verjüngt sich von dem Apex A in Richtung des Vorderendes 10 des lamellenförmigen Arbeitsabschnitts 4. Zumindest einige der Schneidelemente 8, 9, 9.2 des lamellenförmigen Arbeitsabschnitts 4 oder des ersten Abschnitts 7A sind auf dem sich in Richtung des Vorderendes 10 verjüngenden Abschnitt angeordnet. Insbesondere weisen zumindest einige dieser Schneidelemente 8, 9, 9.2 freie Enden, zum Beispiel Spitzen 9B, auf, die im Wesentlichen auf einer ersten Geraden 14 angeordnet sind, wobei die erste Gerade 14 gewinkelt in einem Winkel W1 zur Längsachse 15 des lamellenförmigen Arbeitsabschnitts 4 angeordnet ist. Der Winkel W1 beträgt zum Beispiel in etwa zwischen 7° - 25°, vorzugsweise etwa zwischen 10° - 20°, insbesondere in etwa 12°.

Des Weiteren verjüngt sich die Breite des lamellenförmigen Arbeitsabschnitts 4 von dem Apex A in Richtung des Schafts 5. Zumindest einige der Schneidelemente 8, 9, 9.3 des lamellenförmigen Arbeitsabschnitts 4 sind auf dem sich in Richtung des Schafts 5 verjüngenden Abschnitt angeordnet. Insbesondere weisen zumindest einige dieser Schneidelemente 8, 9, 9.3 freie Enden, zum Beispiel Spitzen 9B, auf, die im Wesentlichen auf einer zweiten Geraden 16 angeordnet sind, wobei die zweite Gerade 16 gewinkelt in einem Winkel W2 zur Längsachse 15 des lamellenförmigen Arbeitsabschnitts 4 angeordnet ist. Der Winkel W2 beträgt zum Beispiel in etwa zwischen 10° - 45°, vorzugsweise etwa zwischen 15° - 30°, insbesondere in etwa 22°.Vorzugsweise sind die erste Gerade 14 und die zweite Gerade 16 gewinkelt zueinander in einem Winkel W3 angeordnet. Der Winkel W3 beträgt zum Beispiel in etwa zwischen 20° - 50°, vorzugsweise etwa zwischen 30° - 40°, insbesondere in etwa 35°.

Zumindest ein Abschnitt des Außenrands des lamellenförmigen Arbeitsabschnitts 4 oder der Umfangskante 7, insbesondere zumindest ein Teil des ersten Abschnitts 7A mit den Schneidelementen 8, 9, zwischen dem Apex A und dem Vorderende 10 des Schneidwerkzeugs 1 ist konkav ausgebildet. Des Weiteren verjüngt sich der lamellenförmigen Arbeitsabschnitt 4 oder die Umfangskante 7, insbesondere zumindest ein Teil des zweiten Abschnitts 7B ohne Schneidelemente, von dem Apex A in Richtung des Schafts 5. Besonders bevorzugt ist zumindest ein Abschnitt des Außenrands des lamellenförmigen Arbeitsabschnitts 4 oder der Umfangskante 7, insbesondere zumindest ein Teil des zweiten Abschnitts 7B ohne Schneidelemente, zwischen dem Apex A und dem Schaft 5 des Schneidwerkzeugs 1 konvex ausgebildet.

Die Figur 5 zeigt eine medizinische, insbesondere dentale, Behandlungsvorrichtung 12 mit einem Handstück 17, an dessen Vorderende das Werkzeug 1 mittels einer Werkzeughaltevorrichtung lösbar befestigt ist. In dem Handstück 17 ist eine (Ultra-)Schallquelle 3 vorgesehen, insbesondere eine piezokeramische Ultraschallquelle. Über einen Schlauch oder eine Leitung 18 ist das Handstück 17 mit einer Steuer- und / oder Regelvorrichtung 19 verbunden. Die Steuer- und / oder Regelvorrichtung 19 steuert und / oder regelt zum Beispiel den Betrieb der Schallquelle 3. An der Steuer- und / oder Regelvorrichtung 19 sind bevorzugt eine Anzeige 20 zum Anzeigen betriebsrelevanter Daten oder zumindest ein Stellelement 21 zum Auswählen oder Einstellen von Betriebsparametern vorgesehen. Eine, vorzugsweise an der Steuer- und / oder Regelvorrichtung 19 befestigte, Flüssigkeitsquelle 22 stellt eine, insbesondere sterile, Behandlungsflüssigkeit zur Verfügung. Eine in der Steuer- und / oder Regelvorrichtung 19 vorgesehene Pumpe fördert die Behandlungsflüssigkeit durch den Schlauch 18 und durch eine Flüssigkeitsleitung in dem Handstück 17 in die Flüssigkeitsleitung 6 des Werkzeugs 1.

Die Figur 6 zeigt ein Ausführungsbeispiel eines Zahns 9, insbesondere eines Zahns 9.1, 9.2, 9.3, des Schneidwerkzeugs 1 oder des lamellenförmigen Arbeitsabschnitts 4 oder des ersten Abschnitts 7A. Der Zahn 9 weist eine Basis 9A, welche ihn mit dem lamellenförmigen Arbeitsabschnitt 4 verbindet, eine Spitze 9B, eine erste Seitenfläche 9C, welche durch die erste Seitenfläche 4A des lamellenförmigen Arbeitsabschnitts 4 gebildet ist, eine zweite Seitenfläche 9D, welche durch die zweite Seitenfläche 4B des lamellenförmigen Arbeitsabschnitts 4 gebildet ist, und zwischen den Seitenflächen 9C, 9D des Zahns 9 angeordnete Schneidflächen 9E1, 9E2 auf. Alternativ setzt sich die erste Seitenfläche 4A des lamellenförmigen Arbeitsabschnitts 4 in der ersten Seitenfläche 9C des Zahns 9 und die zweite Seitenfläche 4B des lamellenförmigen Arbeitsabschnitts 4 in der zweiten Seitenfläche 9D des Zahns 9 fort. Die Schneidflächen 9E1, 9E2 haben eine erste Neigung von der Basis 9A in Richtung der Spitze 9B. Insbesondere vereinigen sich die beiden Schneidflächen 9E1, 9E2 in der Spitze 9B.

Zumindest eine Schneidfläche 9E1, 9E2 hat ausgehend von einer Seitenflächen 9C, 9D des Zahns 9 in Richtung der anderen Seitenflächen 9C, 9D des Zahns 9 (in Bezug auf die Seitenflächen 9C, 9D) eine zweite Neigung. Alternativ oder zusätzlich hat zumindest eine Schneidfläche 9E1, 9E2 ausgehend von einer Seitenflächen 4A, 4B des lamellenförmigen Arbeitsabschnitts 4 in Richtung der anderen Seitenflächen 4A, 4B des lamellenförmigen Arbeitsabschnitts 4 (in Bezug auf die Seitenflächen 4A, 4B) eine zweite Neigung. Diese zweite Neigung kann zum Beispiel eine, insbesondere konkave, Biegung und / oder in Bezug auf die Seitenflächen 4A, 4B und / oder 9C, 9D Abschnitte der Schneidfläche 9E1, 9E2 mit unterschiedlichen Neigungswinkeln umfassen. Die zweite Neigung ist somit derart beschaffen, dass die zumindest eine Schneidfläche 9E1, 9E2 oder zumindest ein Abschnitt davon in einem von einem rechten Winkel abweichenden Winkel zur den Seitenflächen 9C, 9D angeordnet ist. Die Abweichung von dem rechten Winkel beträgt zum Beispiel etwa 1° - 5°, bevorzugt etwa 1° - 3,5°.

Vorzugsweise sind die Flächenmaße der Seitenflächen 9C, 9D eines Zahns 9 unterschiedlich und / oder die Breite der Seitenflächen 9C, 9D eines Zahns 9 sind (parallel zur Basis 9A und im selben Abstand von der Basis 9A des Zahns 9) unterschiedlich. Vorzugsweise ist die zweite Neigung der zumindest einen Schneidfläche 9E1, 9E2 derart beschaffen, dass die Ebenen, in denen die Schneidflächen 9E1, 9E2 angeordnet sind, einander außerhalb des lamellenförmigen Arbeitsabschnitts 4 treffen oder schneiden.

Vorzugsweise wird der zumindest eine Zahn 9 mit der zweiten Neigung oder die zweite Neigung mittels Laserbearbeitung, insbesondere mittels Laserschneiden, hergestellt.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Ein durch Schall antreibbares, medizinisches, insbesondere dentales, Schneidwerkzeug (1), umfassend: Ein Anschlussteil (2) zur Verbindung des Schneidwerkzeugs (1) mit einer Schallquelle (3), einen lamellenförmigen Arbeitsabschnitt (4) und einen den Anschlussteil (2) mit dem lamellenförmigen Arbeitsabschnitt (4) verbindenden Schaft (5), wobei sich eine Flüssigkeitsleitung (6) durch den Schaft (5) bis zu dem lamellenförmigen Arbeitsabschnitt (4) erstreckt und wobei der lamellenförmige Arbeitsabschnitt (4) zwei im Wesentlichen parallel zueinander angeordnete Seitenflächen (4A, 4B) und eine die zwei Seitenflächen (4A, 4B) verbindende Umfangskante (7) aufweist, wobei an der Umfangskante (7) mehrere Schneidelemente (8) in der Form von Zähnen (9) vorgesehen sind, wobei zumindest ein Zahn (9) eine Basis (9A), welche ihn mit dem lamellenförmigen Arbeitsabschnitt (4) verbindet, eine Spitze (9B), eine erste Seitenfläche (9C), welche durch die erste Seitenfläche (4A) des lamellenförmigen Arbeitsabschnitts (4) gebildet ist, eine zweite Seitenfläche (9D), welche durch die zweite Seitenfläche (4B) des lamellenförmigen Arbeitsabschnitts (4) gebildet ist, und zwischen den Seitenflächen (9C, 9D) des Zahns (9) angeordnete Schneidflächen (9E1, 9E2) aufweist, wobei die Schneidflächen (9E1, 9E2) eine erste Neigung von der Basis (9A) in Richtung der Spitze (9B) aufweisen, **dadurch gekennzeichnet, dass**
zumindest eine Schneidfläche (9E1, 9E2) ausgehend von einer Seitenflächen (9C, 9D) des Zahns (9) in Richtung der anderen Seitenflächen (9C, 9D) des Zahns (9) eine zweite Neigung aufweist, so dass die zumindest eine Schneidfläche (9E1, 9E2) in einem von einem rechten Winkel abweichenden Winkel zu den Seitenflächen (9C, 9D) angeordnet ist, wobei der lamellenförmige Arbeitsabschnitt (4) über einen Verbindungsabschnitt (11) mit dem Schaft (5) verbunden ist, wobei der Verbindungsabschnitt (11) eine in Richtung des Schafts (5) zunehmende Außenabmessung aufweist und wobei die Seitenflächen (4A, 4B) des lamellenförmigen Arbeitsabschnitts (4) sich in dem Verbindungsabschnitt (11) fortsetzen, wobei an dem Verbindungsabschnitt (11) mehrere, zum Beispiel zwei, Öffnungen (13) vorgesehen sind, welche die Enden der Flüssigkeitsleitung (6) bilden und durch die Flüssigkeit aus der Flüssigkeitsleitung (6) auf den lamellenförmigen Arbeitsabschnitt (4) und die Behandlungsstelle abgebbar ist, und wobei der lamellenförmige Arbeitsabschnitt (4) einen Teilbereich mit einer größten Breite (B1) aufweist, welcher einen Apex (A) hat, wobei sich die Breite des lamellenförmigen Arbeitsabschnitts (4) von dem Apex (A) in Richtung des Vorderendes (10) des lamellenförmigen Arbeitsabschnitts (4) und in Richtung des Schafts (5) verjüngt, wobei einige der Schneidelemente (8) in Form von Zähnen (9) auf dem sich in Richtung des Vorderendes (10) verjüngenden Abschnitt des lamellenförmigen Arbeitsabschnitts (4) und einige der Schneidelemente (8) in Form von Zähnen (9) auf dem sich in Richtung des Schafts (5) verjüngenden Abschnitt des lamellenförmigen Arbeitsabschnitts (4) angeordnet sind.

2. Schneidwerkzeug (1) nach Anspruch 1, **dadurch gekennzeichnet, dass**
die Umfangskante (7) des lamellenförmigen Arbeitsabschnitts (4) einen ersten Abschnitt (7A) mit Schneidelementen (8) und einen zweiten Abschnitt (7B) ohne Schneidelemente aufweist und dass die Breite der Seitenflächen (4A, 4B) des lamellenförmigen Arbeitsabschnitts (4) zumindest in einem Teilbereich des lamellenförmigen Arbeitsabschnitts (4), insbesondere in dem ersten Abschnitt (7A) mit den Schneidelementen (8), größer ist als der Außendurchmesser (B2) des Schafts (5).

3. Schneidwerkzeug (1) nach Anspruch 2, **dadurch gekennzeichnet, dass**
die Schneidelemente (8) des ersten Abschnitts (7A) mehrere Zähne (9) aufweisen.

4. Schneidwerkzeug (1) nach Anspruch 1, 2 oder 3, **dadurch gekennzeichnet, dass**
an dem lamellenförmigen Arbeitsabschnitt (4) oder an dem ersten Abschnitt (7A) unterschiedliche Zähne (9) vorgesehen sind, die sich zum Beispiel in zumindest einem der folgenden Merkmale unterschieden: Der Größe, der Länge, der Breite oder der Form des Zahns (9), dem Neigungswinkel zumindest einer Zahnflanke, dem Neigungswinkel oder der Neigungsrichtung des gesamten Zahns (9), der Form eines freien Endes des Zahns (9).

5. Schneidwerkzeug (1) nach Anspruch 2, 3 oder 4, **dadurch gekennzeichnet, dass**
die Zähne (9) eine Basis (9A), welche sie mit dem lamellenförmigen Arbeitsabschnitt (4) verbindet, eine Spitze (9B) und eine sich von der Basis (9A) zur Spitze (9B) erstreckende Höhe (H1, H2, H3) haben, wobei an einem Vorderende (10) des ersten Abschnitts (7A) mit den Schneidelementen (8) mehrere erste Zähne (9.1) und an einem daran anschließenden Teilbereich zwischen dem Vorderende (10) des ersten Abschnitts (7A) und dem zweiten Abschnitt (7B) ohne Schneidelemente mehrere zweite Zähne (9.2) vorgesehen sind, wobei die ersten Zähne (9.1) kleiner sind als die zweiten Zähne (9.2) oder wobei die Höhe (H1) der ersten Zähne (9.1) kleiner ist als die Höhe (H2) der zweiten Zähne (9.2).

6. Schneidwerkzeug (1) nach einem der Ansprüche 2 - 5, **dadurch gekennzeichnet, dass**
an einem an den zweiten Abschnitt (7B) ohne Schneidelemente anschließenden Teilbereich des ersten Abschnitts (7A) mit den Schneidelementen (8) mehrere dritte Zähne (9.3) und an einem daran anschließenden Teilbereich des ersten Abschnitts (7A) mehrere zweite Zähne (9.2) vorgesehen sind, wobei die dritten Zähne (9.3) kleiner sind als die zweiten Zähne (9.2) oder wobei die Höhe (H3) der dritten Zähne (9.3) kleiner ist als die Höhe (H2) der zweiten Zähne (9.2).

7. Schneidwerkzeug (1) nach einem der vorstehenden Ansprüche, **dadurch gekennzeichnet, dass**
die Breite der sich in dem Verbindungsabschnitt (11) fortsetzenden Seitenflächen (4A, 4B) des lamellenförmigen Arbeitsabschnitts (4) zumindest in einem Teilbereich des Verbindungsabschnitts (11) größer ist als der Außendurchmesser des Schafts (5).

8. Schneidwerkzeug (1) nach einem der vorstehenden Ansprüche, **gekennzeichnet durch**
ein Anschlussteil (2) mit einer zylindrischen Innenbohrung (2A), wobei an der Wand der Innenbohrung (2A) ein Verbindungselement (2B) zur, insbesondere lösbaren, Verbindung des Schneidwerkzeugs (1) mit der Schallquelle (3) vorgesehen ist.

9. Medizinische, insbesondere dentale, Behandlungsvorrichtung (12), **gekennzeichnet durch**
ein durch Schall antreibbares, medizinisches, insbesondere dentales, Schneidwerkzeug (1) nach einem der vorherstehenden Ansprüche und durch eine Schallquelle (3), wobei das Schneidwerkzeug (1) mit der Schallquelle (3) verbindbar und durch die Schallquelle (3) in Schwingung versetzbar ist.

10. Verfahren zur Herstellung eines durch Schall antreibbaren, medizinischen, insbesondere dentalen, Schneidwerkzeugs (1) nach einem der vorherstehenden Ansprüche, **dadurch gekennzeichnet, dass**
zumindest ein Zahn (9) geformt wird, der eine Basis (9A), welche ihn mit dem lamellenförmigen Arbeitsabschnitt (4) verbindet, eine Spitze (9B), eine erste Seitenfläche (9C), welche durch die erste Seitenfläche (4A) des lamellenförmigen Arbeitsabschnitts (4) gebildet ist, eine zweite Seitenfläche (9D), welche durch die zweite Seitenfläche (4B) des lamellenförmigen Arbeitsabschnitts (4) gebildet ist, und zwischen den Seitenflächen (9C, 9D) des Zahns (9) angeordnete Schneidflächen (9E1, 9E2) aufweist, wobei die Schneidflächen (9E1, 9E2) eine erste Neigung von der Basis (9A) in Richtung der Spitze (9B) aufweisen, und wobei zumindest eine Schneidfläche (9E1, 9E2) ausgehend von einer Seitenflächen (4A, 4B) des lamellenförmigen Arbeitsabschnitts (4) in Richtung der anderen Seitenflächen (4A, 4B) des lamellenförmigen Arbeitsabschnitts (4) eine zweite Neigung aufweist, so dass die zumindest eine Schneidfläche (9E1, 9E2) in einem von einem rechten Winkel abweichenden Winkel zu den Seitenflächen (9C, 9D) angeordnet ist.

11. Verfahren zur Herstellung eines Schneidwerkzeugs (1) nach Anspruch 10, **dadurch gekennzeichnet, dass**
die Schneidelemente (8) oder Zähne (9) durch Laserbearbeitung geformt werden.

12. Verfahren zur Herstellung eines Schneidwerkzeugs (1) nach Anspruch 10 oder 11, **dadurch gekennzeichnet, dass**
die Umfangskante (7) mit einem ersten Abschnitt (7A) mit Schneidelementen (8) und einem zweiten Abschnitt (7B) ohne Schneidelemente derart geformt wird, dass die Breite (B1) der Seitenflächen (4A, 4B) des lamellenförmigen Arbeitsabschnitts (4) zumindest in einem Teilbereich des lamellenförmigen Arbeitsabschnitts (4), insbesondere in dem ersten Abschnitt (7A) mit den Schneidelementen (8), größer ist als der Außendurchmesser (B2) des Schafts (5).

13. Verfahren zur Herstellung eines Schneidwerkzeugs (1) nach Anspruch 12, **dadurch gekennzeichnet, dass**
an dem Vorderende (10) des ersten Abschnitts (7A) mit den Schneidelementen (8) mehrere erste Zähne (9.1) und an einem daran anschließenden Teilbereich zwischen dem Vorderende (10) des ersten Abschnitts (7A) und dem zweiten Abschnitt (7B) ohne Schneidelemente mehrere zweite Zähne (9.2) geformt werden, wobei die ersten Zähne (9.1) kleiner sind als die zweiten Zähne (9.2) oder wobei die Höhe (H1) der ersten Zähne (9.1) kleiner ist als die Höhe (H2) der zweiten Zähne (9.2).

## Claims

1. A medical, in particular dental, cutting tool (1) which is drivable by sound, comprising:
a connecting part (2) for connecting the cutting tool (1) to a sound source (3), a lamellar working section (4) and a shaft (5) connecting the connecting part (2) to the lamellar working section (4), wherein a liquid line (6) extends through the shaft (5) up to the lamellar working section (4) and wherein the lamellar working section (4) comprises two side faces (4A, 4B) arranged essentially parallel to one another and a peripheral edge (7) connecting the two side faces (4A, 4B), wherein a plurality of cutting elements (8) in the form of teeth (9) are provided on the peripheral edge (7), wherein at least one tooth (9) comprises a base (9A) connecting the latter to the lamellar working section (4), a tip (9B), a first side face (9C) formed by the first side face (4A) of the lamellar working section (4), a second side face (9D) formed by the second side face (4B) of the lamellar working section (4), and cutting faces (9E1, 9E2) arranged between the side faces (9C, 9D) of the tooth (9), wherein the cutting faces (9E1, 9E2) have a first inclination from the base (9A) in the direction of the tip (9B), **characterized in that**
at least one cutting face (9E1, 9E2) has a second inclination starting from one side face (9C, 9D) of the tooth (9) in the direction of the other side faces (9C, 9D) of the tooth (9), so that the at least one cutting face (9E1, 9E2) is arranged at an angle deviating from a right angle to the side faces (9C, 9D), wherein the lamellar working section (4) is connected to the shaft (5) via a connecting section (11), wherein the connecting section (11) comprises an outside dimension that increases in the direction of the shaft (5) and wherein the side faces (4A, 4B) of the lamellar working section (4) are continued in the connecting section (11), wherein a plurality of openings (13), for example, two openings, are provided on the connecting section (11) which form the ends of the liquid line (6) and through which fluid from the fluid line (6) is dispensable onto the lamellar working section (4) and the treatment site, and wherein the lamellar working section (4) has a portion with a largest width (B1) which has an apex (A), wherein the width of the lamellar working section (4) tapers from the apex (A) in the direction of the front end (10) of the lamellar working section (4) and in the direction of the shaft (5), wherein some of the cutting elements (8) in the form of teeth (9) are arranged on the section of the lamellar working section (4) that tapers in the direction of the front end (10), and some of the cutting elements (8) in the form of teeth (9) are arranged on the section of the lamellar working section (4) that tapers in the direction of the shaft (5).

2. The cutting tool (1) according to claim 1, **characterized in that** the peripheral edge (7) of the lamellar working section (4) comprises a first section (7A) with cutting elements (8) and a second section (7B) without cutting elements, and that the width of the side faces (4A, 4B) of the lamellar working section (4) is greater than the outside diameter (B2) of the shaft (5) in at least a portion of the lamellar working section (4), in particular in the first section (7A) with the cutting elements (8).

3. The cutting tool (1) according to claim 2, **characterized in that**
the cutting elements (8) of the first section (7A) comprise a plurality of teeth (9).

4. The cutting tool (1) according to claim 1, 2 or 3, **characterized in that**
different teeth are provided on the lamellar working section (4) or on the first section (7A), differing in at least one of the following features: the size, length, width or shape of the tooth (9), the angle of inclination of at least one tooth flank, the angle of inclination or the direction of inclination of the entire tooth (9), the shape of a free end of the tooth (9).

5. The cutting tool (1) according to claim 2, 3 or 4, **characterized in that**
the teeth (9) comprise a base (9A) connecting them to the lamellar working section (4), at tip (9B) and a height (H1, H2, H3) extending from the base (9A) to the tip (9B), wherein a plurality of first teeth (9.1) are provided on a front end (10) of the first section (7A) with the cutting elements (8), and a plurality of second teeth (9.2) are provided on a following portion between the front end (10) of the first section (7A) and the second section (7B) without cutting elements, wherein the first teeth (9.1) are smaller than the second teeth (9.2) or wherein the height (H1) of the first teeth (9.1) is less than the height (H2) of the second teeth (9.2).

6. The cutting tool (1) according to any one of claims 2 - 5, **characterized in that**
a plurality of third teeth (9.3) are provided on a portion of the first section (7A) with cutting elements (8) following the second section (7B) without cutting elements, and a plurality of second teeth (9.2) are provided on a following portion of the first section (7A), wherein the third teeth (9.3) are smaller than the second teeth (9.2) or wherein the height (H3) of the third teeth (9.3) is less than the height (H2) of the second teeth (9.2).

7. The cutting tool (1) according to any one of the preceding claims, **characterized in that**
the width of the side faces (4A, 4B) of the lamellar working section (4) which are continued in the connection section (11) is greater in at least a portion of the connection section (11) than the outside diameter of the shaft (5).

8. The cutting tool (1) according to any one of the preceding claims, **characterized by**
a connecting part (2) with a cylindrical inside bore (2A), wherein a connecting element (2B) is provided on the wall of the inside bore (2A), in particular for a releasable connection of the cutting tool (1) with the sound source (3).

9. A medical, in particular dental, treatment device (12), **characterized by**
a medical, in particular dental, cutting tool (1) drivable by sound according to any one of the preceding claims and by a sound source (3), wherein the cutting tool (1) is connectable to the sound source (3) and can be set in vibration by the sound source (3).

10. A method for producing a medical, in particular dental, cutting tool (1) that is drivable by sound according to any one of the preceding claims, **characterized in that** at least one tooth (9) is shaped comprising a base (9A) which connects the tooth to the lamellar working section (4), a tip (9B), a first side face (9C) which is formed by the first side face (4A) of the lamellar working section (4), and a second side face (9D) which is formed by the second side face (4B) of the lamellar working section (4), and cutting faces (9E1, 9E2) arranged between the side faces (9C, 9D) of the tooth (9), wherein the cutting faces (9E1, 9E2) have a first inclination from the base (9A) in the direction of the tip (9B) and wherein at least one cutting face (9E1, 9E2) has a second inclination starting from the side faces (4A, 4B) of the lamellar working section (4) in the direction of the other side faces (4A, 4B) of the lamellar working section (4) so that the at least one cutting face (9E1, 9E2) is arranged at an angle to the side faces (9C, 9D) which deviates from the right angle.

11. The method for manufacturing a cutting tool (1) according to claim 10, **characterized in that**
the cutting elements (8) or teeth (9) are shaped by laser machining.

12. The method for manufacturing a cutting tool (1) according to claim 10 or 11, **characterized in that**
the peripheral edge (7) with a first section (7A) with cutting elements (8) and a second section (7B) without cutting elements is shaped such that the width (B1) of the side faces (4A, 4B) of the lamellar working section (4) is larger in at least a portion of the lamellar working section (4), in particular in the first section (7A) with the cutting elements (8) than the outside diameter (B2) of the shaft (5).

13. The method for manufacturing a cutting tool (1) according to claim 12, **characterized in that**
a plurality of first teeth (9.1) are arranged on the front end (10) of the first section (7A) with the cutting elements (8) and a plurality of second teeth (9.2) are formed on a following portion between the front end (10) of the first section (7A) and the second section (7B) without cutting elements, wherein the first teeth (9.1) are smaller than the second teeth (9.2) or wherein the height (H1) of the first teeth (9.1) is smaller than the height (H2) of the second teeth (9.2).

## Revendications

1. Outil de coupe médical, en particulier dentaire, pouvant être entraîné par le son (1), comprenant: une pièce de raccordement (2) destinée à relier l'outil de coupe (1) à une source de son (3), une section opératoire en forme de lamelle (4) et un arbre (5) reliant la pièce de raccordement (2) à la section opératoire en forme de lamelle (4), une conduite de liquide (6) s'étendant dans l'arbre (5) jusqu'à la section opératoire en forme de lamelle (4) et la section opératoire en forme de lamelle (4) présentant deux surfaces latérales (4A, 4B) s'étendant sensiblement à la parallèle l'une de l'autre (4A, 4B) et une arête circonférentielle (7) reliant les deux surfaces latérales (4A, 4B), plusieurs éléments de coupe (8) se présentant sous forme de dents (9) étant prévus au niveau de l'arête circonférentielle (7), au moins une dent (9) présentant une base (9A), qui la relie à la section opératoire en forme de lamelle (4), une pointe (9B), une première surface latérale (9C) qui est constituée par la première surface latérale (4A) de la section opératoire en forme de lamelle (4), une deuxième surface latérale (9D) qui est constituée par la deuxième surface latérale (4B) de la section opératoire en forme de lamelle (4) et des surfaces de coupe (9E1, 9E2) disposées entre les surfaces latérales (9C, 9D) de la dent (9), les surfaces de coupe (9E1, 9E2) présentant une première inclinaison depuis la base (9A) en direction de la pointe (9B), **caractérisée en ce**
**qu'**au moins une surface de coupe (9E1, 9E2), en partant d'une des surfaces latérales (9C, 9D) de la dent (9), présente, en direction des autres surfaces latérales (9C, 9D) de la dent (9), une deuxième inclinaison, de sorte que l'au moins une surface de coupe (9E1, 9E2) est disposée suivant un angle différent d'un angle droit par rapport aux surfaces latérales (9C, 9D), la section opératoire en forme de lamelle (4) étant reliée par une section de liaison (11) à l'arbre (5), la section de liaison (11) présentant une dimension extérieure augmentant en direction de l'arbre (5) et les surfaces latérales (4A, 4B) de la section opératoire en forme de lamelle (4) se continuant dans la section de liaison (11), étant prévues au niveau de la section de liaison (11) plusieurs, par exemple deux ouvertures (13) qui constituent les extrémités de la conduite de liquide (6) et à travers desquels le liquide provenant de la conduite de liquide (6) peut être distribué sur la section opératoire en forme de lamelle (4) et le point de traitement et la section opératoire en forme de lamelle (4) présentant une sous-partie ayant une largeur maximale (B1) qui comporte un apex (A), la largeur de la section opératoire en forme de lamelle (4) se rétrécissant depuis l'apex (A) en direction de l'extrémité avant (10) de la section opératoire en forme de lamelle (4) et en direction de l'arbre (5), certains des éléments de coupe (8) se présentant sous forme de dents (9) étant disposés sur la section se rétrécissant en direction de l'extrémité avant (10) de la section opératoire en forme de lamelle (4) et certains des éléments de coupe (8) se présentant sous forme de dents (9) étant disposés sur la section opératoire se rétrécissant en direction de l'arbre (5) de la section opératoire en forme de lamelle (4).

2. Outil de coupe (1) selon la revendication 1, **caractérisé en ce que**
l'arête circonférentielle (7) de la section opératoire en forme de lamelle (4) présente une première section (7A) dotée d'éléments de coupe (8) et une deuxième section (7B) sans éléments de coupe et que la largeur des surfaces latérales (4A, 4B) de la section opératoire en forme de lamelle (4) est, du moins dans une sous-partie de la section opératoire en forme de lamelle (4), en particulier dans la première section (7A) dotée d'éléments de coupe (8), supérieure au diamètre extérieur (B2) de l'arbre (5).

3. Outil de coupe (1) selon la revendication 2, **caractérisé en ce que**
les éléments de coupe (8) de la première section (7A) présentent plusieurs dents (9).

4. Outil de coupe (1) selon la revendication 1, 2 ou 3, **caractérisé en ce que**,
au niveau de la section opératoire en forme de lamelle (4) ou au niveau de la première section (7A), il est prévu des dents différentes (9) qui se différencient par exemple par au moins une des caractéristiques suivantes: la taille, la longueur, la largeur ou la forme de la dent (9), l'angle d'inclinaison d'au moins un flanc de la dent, l'angle d'inclinaison ou le sens d'inclinaison de l'ensemble de la dent (9), la forme d'une extrémité libre de la dent (9).

5. Outil de coupe (1) selon la revendication 2, 3 ou 4, **caractérisé en ce que**
les dents (9) comportent une base (9A) qui les relie à la section opératoire en forme de lamelle (4), une pointe (9B) et ont une hauteur (H1, H2, H3) s'étendant de la base (9A) à la pointe (9B), étant prévues à une extrémité avant (10) de la première section (7A) dotée d'éléments de coupe (8), plusieurs premières dents (9.1) et, au niveau d'une sous-partie s'y raccordant entre l'extrémité avant (10) de la première section (7A) et la deuxième section (7B) sans éléments de coupe, plusieurs deuxièmes dents (9.2), les premières dents (9.1) étant plus petites que les deuxièmes dents (9.2) ou la hauteur (H1) des premières dents (9.1) étant inférieure à la hauteur (H2) des deuxièmes dents (9.2).

6. Outil de coupe (1) selon une des revendications 2 à 5, **caractérisé en ce que**,
au niveau d'une sous-partie de la première section (7A) dotée des éléments de coupe (8) se raccordant à la deuxième section (7B) sans éléments de coupe, il est prévu plusieurs troisièmes dents (9.3) et au niveau d'une sous-partie de la première section (7A) s'y raccordant, plusieurs deuxièmes dents (9.2), les troisièmes dents (9.3) étant plus petites que les deuxièmes dents (9.2) ou la hauteur (H3) des troisièmes dents (9.3) étant inférieure à la hauteur (H2) des deuxièmes dents (9.2).

7. Outil de coupe (1) selon une des revendications précédentes, **caractérisé en ce que** la largeur des surfaces latérales (4A, 4B) de la section opératoire en forme de lamelle (4) qui se continuant dans la section de liaison (11) est, du moins dans une sous-partie de la section de liaison (11), supérieure au diamètre extérieur de l'arbre (5).

8. Outil de coupe (1) selon une des revendications précédentes, **caractérisé par**
une pièce de raccordement (2) dotée d'un alésage cylindrique interne (2A), étant prévu, au niveau de la paroi de l'alésage interne (2A), un élément de liaison (2B) destiné au raccordement, en particulier dissociable, de l'outil de coupe (1) à la source de son (3).

9. Dispositif de traitement médical, en particulier dentaire (12), **caractérisé par** un outil de coupe médical, en particulier dentaire, pouvant être entraîné par le son (1) selon une des revendications précédentes et par une source de son (3), l'outil de coupe (1) pouvant être raccordé à la source de son (3) et être mis en vibration par la source de son (3).

10. Procédé de fabrication d'un outil de coupe médical, en particulier dentaire, pouvant être entraîné par le son (1) selon une des revendications précédentes, **caractérisé en ce**
**qu'**il est formé au moins une dent (9) qui présente une base (9A) qui la relie à la section opératoire en forme de lamelle (4), une pointe (9B), une première surface latérale (9C) qui est constituée par la première surface latérale (4A) de la section opératoire en forme de lamelle (4), une deuxième surface latérale (9D) qui est constituée par la deuxième surface latérale (4B) de l'élément opératoire en forme de lamelle (4) et des surfaces de coupe (9E1, 9E2) disposées entre les surfaces latérales (9C, 9D) de la dent (9), les surfaces de coupe (9E1, 9E2) présentant une première inclinaison depuis la base (9A) en direction de la pointe (9B) et au moins une surface de coupe (9E1, 9E2), en partant d'une des surfaces latérales (4A, 4B) de l'élément opératoire en forme de lamelle (4) présentant, en direction des autres surfaces latérales (4A, 4B) de l'élément opératoire en forme de lamelle (4), une deuxième inclinaison, de sorte que l'au moins une surface de coupe (9E1, 9E2) est disposée suivant un angle différent d'un angle droit par rapport aux surfaces latérales (9C, 9D).

11. Procédé de fabrication d'un outil de coupe (1) selon la revendication 10, **caractérisé en ce que**
les éléments de coupe (8) ou les dents (9) sont formés par usinage au laser.

12. Procédé de fabrication d'un outil de coupe (1) selon la revendication 10 ou 11, **caractérisé en ce que**
l'arête circonférentielle (7) est formée avec une première section (7A) dotée d'éléments de coupe (8) et une seconde section (7B) sans éléments de coupe de manière à ce que la largeur (B1) des surfaces latérales (4A, 4B) de la section opératoire en forme de coupe (4) soit, du moins dans une sous-partie de la section opératoire en forme de lamelle (4), en particulier dans la première section (7A) doté des éléments de coupe (8), supérieure au diamètre extérieur (B2) de l'arbre (5).

13. Procédé de fabrication d'un outil de coupe (1) selon la revendication 12, **caractérisé en ce que**,
à l'extrémité avant (10) de la première section (7A) dotée des éléments de coupe (8), plusieurs premières dents (9.1) et, au niveau d'une sous-partie s'y raccordant entre l'extrémité avant (10) de la première section (7A) et la deuxième section (7B) sans éléments de coupe, plusieurs deuxièmes dents (9.2) sont formées, les premières dents (9.1) étant plus petites que les deuxièmes dents (9.2) ou la hauteur (H1) des premières dents (9.1) étant inférieure à la hauteur (H2) des deuxièmes dents (9.2).
